Europäisches Patentamt

European Patent Office

Office européen des brevets

(11) Veröffentlichungsnummer : **0 113 926
B1**

(12) **EUROPÄISCHE PATENTSCHRIFT**

(45) Veröffentlichungstag der Patentschrift :
12.11.86

(51) Int. Cl.⁴ : **C 09 C 1/30**

(21) Anmeldenummer : **83113266.7**

(22) Anmeldetag : **31.12.83**

(54) **Verwendung oberflächenverkleinerter Kieselgele als Füllstoff für Dentalmassen.**

(30) Priorität : **07.01.83 DE 3300321**

(43) Veröffentlichungstag der Anmeldung :
**25.07.84 Patentblatt 84/30**

(45) Bekanntmachung des Hinweises auf die Patenterteilung : **12.11.86 Patentblatt 86/46**

(84) Benannte Vertragsstaaten :
**AT BE CH DE FR GB IT LI LU NL SE**

(56) Entgegenhaltungen :
**FR-A- 2 246 497
GB-A- 866 326**

(73) Patentinhaber : **Ernst Mühlbauer KG
Fangdieckstrasse 61
D-2000 Hamburg 53 (DE)**

(72) Erfinder : **Engelbrecht, Jürgen, Dr., Dipl.-Chem.
Petkumstrasse 18
D-2000 Hamburg 76 (DE)**
Erfinder : **Günther, Michael, Dr., Dipl.-Chem.
Edvard-Munch-Strasse 39
D-2000 Hamburg 74 (DE)**
Erfinder : **von Wallis, Helmut, Dr., Dipl.-Chem.
Hirschberger Strasse 2
D-2359 Henstedt-Ulzburg 2 (DE)**

(74) Vertreter : **Wolff, Hans Joachim, Dr.jur. Dipl.-Chem. et al
Beil, Wolff & Beil Rechtsanwälte Postfach 80 01 40
Adelonstrasse 58
D-6230 Frankfurt am Main 80 (DE)**

**Beschreibung**

Die Erfindung betrifft die Verwendung eines oberflächenverkleinerten Kieselgels als Füllstoff für Dentalmassen.

Dentalmaterialien auf der Basis polymerisierbarer Verbindungen enthalten neben einem oder mehreren polymerisierbaren Monomeren, Polymerisationskatalysatoren, Beschleunigern, UV-Stabilisatoren usw. einen in der Regel anorganischen Füllstoff, der zur Verringerung des Polymerisationsschrumpfes, Erniedrigung des thermischen Expansionskoeffizienten, Verringerung der Wasseraufnahme und Erhöhung der Härte der erhaltenen Polymerisate zugesetzt wird.

Hierbei gibt es zum einen die Gruppe der sogenannten « Makrofiller », d. h. Füllstoffe mit einem Teilchendurchmesser von 0,1 bis 100 µm. Derartige Stoffe sind : gemahlener Quarz, Quarz- oder Silikatgläser, Aluminiumoxid, keramische oder mineralische Füllstoffe. Diese lassen sich in relativ großen Mengen in Dentalmassen einarbeiten. So sind sie z. B. in « Composite »-Materialien in einer Menge von etwa 60 bis 80 Gew.-% der Gesamtzusammensetzung enthalten. Je höher der Anteil an anorganischem Füllstoff, desto besser sind die physikalischen Eigenschaften des auspolymerisierten Materials, wie geringer Expansionskoeffizient, geringe Wasseraufnahme und Farbstabilität. Je höher der Anteil an diesen « Makrofillern », desto schlechter ist jedoch die Polierbarkeit der auspolymerisierten Kunststoffe.

Die Oberflächenrauhigkeit der erhaltenen Materialien führt zu Problemen, da hierdurch die Ablagerung von Zahnbelag gefördert wird und dadurch sowohl Verfärbungen als auch marginale Sekundärkaries verursacht werden können.

Nachteilig ist ferner die noch nicht befriedigende Abriebfestigkeit obiger Dentalmaterialien. Durch ihr schlechtes Abriebverhalten können die Zahnfüllmaterialien mit den genannten Füllstoffen nicht im Molaren-Gebiet eingesetzt werden, wo trotz des toxikologisch bedenklichen Quecksilbers die Amalgamfüllungen noch vorherrschen.

Um die Polierbarkeit und Abriebfestigkeit solcher Kunststoffe zu verbessern, hat man deshalb zum anderen vorgeschlagen, Füllstoffe mit kleinsten Primärteilchengrößen, sogenannte « Mikrofiller », in dentalen Materialien einzusetzen.

Aus Hirasawa (Reports of the Institute for Medical and Dental Engineering 2, (1986) 55-61) sowie aus der DE-OS 24 03 211 ist bekannt, daß amorphe Kieselsäuren, wie Kieselgel oder pyrogene Kieselsäure mit Primärteilchengrößen von 5-50 nm nach dem Aushärten der damit hergestellten Dentalmassen gut polierbare Formkörper mit günstigen Abrasionseigenschaften ergeben.

Die Verbesserung von Polierbarkeit und Abrasionsfestigkeit geht jedoch einher mit einer Verschlechterung einiger anderer wichtiger Eigenschaften, wie z. B. Wasseraufnahme und Farbstabilität. Hohe Wasseraufnahme und mangelnde Farbstabilität werden gefördert durch die in den Dentalmassen enthaltenen Harze. Man ist deshalb bestrebt, den Harzanteil zu verringern und diese unerwünschten Eigenschaften durch Erhöhung des Anteils an anorganischen Füllstoffen zu beseitigen. Mit den bekannten « Mikroffilern » ist dieses Problem nur unbefriedigend zu lösen, da sie nur zu einem Anteil bis etwa 50 Gew.-% in ein dentales Pastenmaterial einarbeitbar sind. Außerdem muß man bei Verwendung von Mikrofillern einen recht hohen thermischen Expansionskoeffizienten in Kauf nehmen. Wenn Füllungsmaterial und Zahnsubstanz einen stark unterschiedlichen thermischen Expansionskoeffizienten aufweisen, wird durch den z. B. durch die Nahrungsaufnahme bedingten Temperaturwechsel der sogenannte « Pumpeffekt » hervorgerufen. Durch den gebildeten Randspalt können Bakterien eindringen und zu Sekundärkaries oder in tiefen Kavitäten zu Pulpenschädigungen führen.

Es ist versucht worden, diese Nachteile dadurch zu vermeiden, daß man kleinste, im Millimikronenbereich liegende Teilchen von pyrogener und Fällungskieselsäure vornehmlich durch oxidische Bindemittel zu wesentlich größeren im Mikronenbereich liegenden Teilchen agglomerierte und in dieser agglomerierten Form als Füllstoff in Dentalmassen einsetzte, wie in der WO 81/01366 beschrieben. Mit diesen Füllstoffen können Pasten höher gefüllt werden, als mit normalen « Mikrofillern ». Die Formkörper zeigen noch zufriedenstellende Polierbarkeit und Abrasionsbeständigkeit.

Diese Entwicklung stellte zweifellos eine Verbesserung dar. Die Formkörper liegen jedoch in ihren thermischen Expansionskoeffizienten im Vergleich zu dem, was mit « Makrofillern » erreichbar ist, weit unterhalb des Erforderlichen.

Aus der FR-A-2 246 497 ist ein Verfahren zur Herstellung eines Füllstoffs für Dentalmassen bekannt, bei dem ein natürlich vorkommendes Siliciumdioxid einer chemischen Behandlung mit Säure und einer thermischen Behandlung unterworfen wird. Natürlich vorkommendes $SiO_2$ und Silicagel sind jedoch völlig unterschiedliche Materialien und unterscheiden sich in der Hauptsache durch Dichte, Brechungsindex und spezifische Oberfläche und außerdem dadurch, daß natürlich vorkommendes $SiO_2$ so stark verunreinigt ist, daß selbst die säure-wärmebehandelten natürlich vorkommenden $SiO_2$-Materialien nicht rein genug sind, um bei ihrer Verwendung als Füllstoffe ausreichend weiße Dentalmasse zu liefern. Außerdem können die in diesen behandelten Materialien immer noch enthaltenen Verunreinigungen die Polymerisationsinitiatoren in den Dentalmassen beeinträchtigen. Schließlich weicht der Brechungsindex von $SiO_2$ von den Brechungsindices der damit zu kombinierenden Materialien, d. h. Harz und ggf. silanisierendes Material, ab und resultiert in unerwünscht opaken Dentalmassen.

Aufgabe der Erfindung war es daher, einen neuen Füllstoff zu finden, der mit allen seinen Eigenschaften in den Bereichen liegt, die bisher nur durch Anwendung von « Makrofillern » erreicht werden konnten, darüberhinaus jedoch eine ausgezeichnete Polierbarkeit und Abrasionsbeständigkeit aufweist.

Erfindungsgemäß wird dies erreicht durch Verwendung eines oberflächenverkleinerten Kieselgels einer mittleren Teilchengröße von etwa 0,5-50 μm, vorzugsweise 1-20 μm, das dadurch hergestellt wurde, daß man ein übliches Kieselgel etwa der gleichen Korngröße mit größerer spezifischer Oberfläche einer thermischen Behandlung durch einen Glühprozess zwischen 500 und 1 200 °C und/oder einer chemisch-katalytischen Behandlung mit Säure unterwarf, als Füllstoff für Dentalmassen auf Basis polymerisierbarer Monomerer.

Es war völlig überraschend, daß ein Kieselgel mit verringerter Oberfläche nicht nur in großen Mengen in Dentalmassen einarbeitbar ist und denselben eine gute Abriebfestigkeit und Polierbarkeit verleiht, sondern daß diesen Dentalmassen auch ein erstaunlich niedriger Expansionskoeffizient verliehen werden kann.

Es war vor allen Dingen nicht zu erwarten, daß dies durch die Verwendung von Kieselgel zu erreichen ist, da in relevanten Veröffentlichungen die Eignung von Kieselgel als Füllstoff für Dentalmassen noch heute bestritten wird.

Schließlich war es überraschend, daß durch bestimmte Behandlungsmethoden ohne bleibenden Zusatz von Fremdsubstanzen solche verringerten Oberflächen zu erzielen waren.

Die Oberfläche von Silicagel durch thermische Behandlung zu verkleinern, ist generell aus Kirk-Othmer, Encyclopedia of Chemical Technology, 3$^{rd}$ Edition, Vol. 20, S. 774-775 bekannt.

Die thermische Behandlung erfolgt durch längeres Glühen der Pulver bei Temperaturen zwischen 500-1 200 °C, wobei Temperatur und Glühdauer derart gewählt werden müssen, daß einerseits der Sinterprozeß zu ausreichender Oberflächenverkleinerung führt, andererseits jedoch das Kieselgel nicht zu einer glasartigen Masse schmilzt. Bei der chemisch-katalytischen Behandlung wird das Ausgangsmaterial mit Säure gekocht, anschließend gewaschen und getrocknet. Man kann auch beide Verfahren Kombinieren. Die Art der Säure ist nicht kritisch. Es eignen sich praktisch alle stärkeren Säuren. Die Säure sollte sich lediglich gut auswaschen lassen und beim kombinierten Verfahren leicht vertreiben lassen. Ein Beispiel für eine besonders vorteilhafte Säure ist Salzsäure.

Die erfindungsgemäß erhaltenen Kieselgele zeigen eine deutlich verringerte spezifische Oberfläche, sind jedoch in anderen wichtigen und günstigen Eigenschaften, wie z. B. im Brechungsindex, im wesentlichen gleich geblieben.

Eine besonderer Vorteil ist, daß die Korngrößenverteilung in etwa unverändert bleibt und damit ein nachträgliches Mahlen und Sieben im Gegensatz zu dem in WO 81/01366 beschriebenen Verfahren in der Regel entfällt.

Das erfindungsgemäße oberflächenverkleinerte Kieselgel läßt sich gut mit flüssigen polymerisierbaren Monomeren vermischen. Dabei haben sich als Monomere die mono-, di- oder polyfunktionellen Derivate der Acryl- oder Methacrylsäure bewährt, insbesondere die Ester. Der erfindungsgemäße Füllstoff kann dabei gegebenenfalls zusammen mit anderen üblichen anorganischen Füllstoffen, wie z. B. Quarz, amorphem Siliziumdioxid, Gläsern, keramischen oder mineralischen Füllstoffen und/oder organischen ungefüllten oder gefüllten Polymerisaten verwendet werden. Der anorganische Füllstoffanteil in den erfindungsgemäßen Dentalmassen soll zwischen 10 und 80 %, vorzugsweise zwischen 30 und 80 % und besonders bevorzugt zwischen 50 und 70 % bezogen auf die Gesamtmasse betragen.

Das oberflächenverkleinerte Kieselgel kann vor seiner Verwendung auch silanisiert werden, um die Bindung mit dem Polymer zu verbessern, z. B. durch Behandlung mit Trimethoxy-(3-methacryloxypropyl)-silan. Bei Verwendung in Dentalpräparaten wird in der Regel zur Anpassung an die natürlichen Zähne die Masse nocht mit bekannten organischen oder anorganischen Farbpigmenten und/oder Trübungsmitteln versetzt. Als Härtungskatalysatoren können in diesen Präparaten u. a. organische Peroxide, wie Dibenzoylperoxid oder Azo-Verbindungen, wie Azo-bis-iso-butyronitril eingesetzt werden. Auch die für die Kalt-Härtung von Vinyl-ungesättigten Monomeren geeigneten Redoxysteme, wie z. B. Dibenzoylperoxid/N,N-Bis-2-hydroxyäthylxylidin oder Dibenzoyl-peroxid/Barbitursäurederivate sind geeignet. Als Härtungskatalysatoren können aber auch Substanzen eingesetzt werden, die nach Bestrahlung mit UV- oder sichtbarem Licht die Polymerisation auslösen, wie z. B. Benzoin-alkyläther, Benzilmonoketale oder aliphatische und aromatische 1,2-Diketoverbindungen, wobei die Lichtpolymerisation durch Zusätze von Aktivatoren, wie Aminen oder organischen Phosphiten, in an sich bekannter Weise beschleunigt werden kann.

Nach dem Aushärten werden die guten Eigenschaften der klassischen « Makrofiller »-Composites wie geringe thermische Expansion, hohe Farbstabilität und geringe Wasseraufnahme, mit den guten Eigenschaften der vorbekannten mit amorpher Kieselsäure hergestellten Formkörper hinsichtlich Polierbarkeit und Abrasionsfestigkeit in einem Material vereinigt, ohne daß hierbei Nachteile in Kauf genommen werden müssen.

Der erfindungsgemäße Füllstoff eignet sich allgemein für Dentalmassen, also nicht nur für Zahnfüllpräparate, sondern auch für solche Zwecke, für die derartige Werkstoffe üblicherweise vorteilhaft verwendet werden, z. B. zur Herstellung und Reparatur von Kronen, Brücken, Verblendungen und ähnlichen Zahnersatzteilen, zur Herstellung von künstlichen Zähnen oder auch zur Herstellung von Versiegelungs- und Befestigungswerkstoffen.

Herstellung von oberflächenverkleinerten Kieselgelen durch thermische Behandlung.

## Beispiel 1

Kieselgel einer mittleren Korngröße von ca. 2 μm und einer BET-Oberfläche von ca. 400 m²/g wurde bei einer Temperatur von 900 °C 18 h geglüht. Das erkaltete Produkt wurde durch ein 45 μm-Sieb gedrück und hatte eine BET-Oberfläche von ca. 130 m²/g. Korngrößenverteilung und Brechungsindex des erhaltenen Materials waren nahezu unverändert.

## Beispiel 2

Kieselgel einer mittleren Korngröße von ca. 12 μm und einer BET-Oberfläche von ca. 400 m²/g wurde unter gleichen Bedingungen wie im Beispiel 1 behandelt. Das gewonnene Pulver hatte eine BET-Oberfläche von ca. 200 m²/g, zeigte jedoch unverändert eine mittlere Korngröße von ca. 12 μm und einen Brechungsindex von 1,46.

Herstellung von oberflächenverkleinerten Kieselgelen durch chemisch-katalytische Behandlung.

## Beispiel 3

200 g Kieselgel einer mittleren Korngröße von ca. 2 μm und einer BET-Oberfläche von ca. 270 m²/g wurden mit 2,5 l konz. HCl 2 Tage unter Rückfluß gekocht. Nach Filtration und mehrmaligem Waschen mit Wasser wurde bei 200 °C getrocknet und danach gesiebt. Das erhaltene Pulver zeigte eine verminderte BET-Oberfläche von ca. 200 m²/g. Korngrößenverteilung und Brechungsindex waren unverändert.

Lichthärtbares Füllungsmaterial.

## Beispiel 4

Eine Harzmischung wurde hergestellt aus :

285  g Urethandimethacrylat
323  g Hexandioldimethacrylat
380  g Bis-GMA
  4,5 g Dimethoxybenzyl
  2,5 g Campherchinon
  2,5 g Dimethylbutylanilin.

Eine Paste wurde hergestellt aus :

26,7 g obiger Harzmischung
44,4 g Kieselgel nach Beispiel 2 mit einer BET-Oberfläche von 200 m²/g, auf übliche Weise silanisiert
 9,7 g Kieselgel nach Beispiel 1 mit einer BET-Oberfläche von 130 m²/g, auf übliche Weise silanisiert
 1,9 g pyrogene silanisierte Kieselsäure.

Das erhaltene Material zeigte nach Einfärbung mit Pigmenten und Belichten mit einem handelsüblichen Halogenlicht-Bestrahlungsgerät (20 sec.) eine Durchhärtungstiefe von ca. 4 mm und eine ausgezeichnete Transparenz und Zahnähnlichkeit. Bei einem anorganischen Füllstoffgehalt von 68 % war das Material noch hochglanzpolierbar und um ein Vielfaches abrasionsresistenter als Composites aus « Makrofillern ». Es hatte einen erhöhten Biegemodul von 5800 N/mm² und einen linearen thermischen Expansionskoeffizienten von nur 28 ppm/°C.

Die Wasseraufnahme war gegenüber einem Füllmaterial aus handelsüblichem Kieselgel auf 0,42 mg/cm² erniedrigt (siehe Vergleichsbeispiel 1). Es eignete sich sehr gut als Zahnfüllungsmaterial im Molaren-Bereich.

## Vergleichsbeispiel 1

Vergleich mit handelsüblichem Kieselgel.
Eine Paste wurde hergestellt aus :

80,7 g Harzmischung aus Beispiel 4
44,4 g Kieselgel mit einer BET-Oberfläche von 400 m²/g, mittlere Teilchengröße 12 μm, silanisiert
 9,7 g Kieselgel, mittlere BET-Oberfläche von 400 m²/g, mittlere Teilchengröße 2 μm, silanisiert
 1,9 g pyrogene Kieselsäure, silanisiert.

Das erhaltene Material zeigte ebenfalls nach Pigmentierung und Aushärtung durch Belichten eine gute Transparenz und Zahnähnlichkeit, Hochglanzpolierbarkeit und Abrasionsresistenz. Bei einem anorganischen Füllstoffgehalt von nur 41 Gew.-% betrug der Biegemodul jedoch nur 2 700 N/mm², der lineare thermische Ausdehnungskoeffizient 64 ppm/°C und die Wasseraufnahme 0,81 mg/cm².

Um eine hinsichtlich der Konsistenz mit der erfindungsgemäßen Paste gemäß Beispiel 4 vergleichbare Paste zu erhalten, konnte nicht mehr als die oben angegebene Menge Kieselgel eingearbeitet werden.

Vergleichsbeispiel 2

Das erfindungsgemäße dentale Füllungsmaterial I nach Beispiel 4 wird mit dem Material II nach Vergleichsbeispiel 1 sowie mit drei handelsüblichen Füllungsmaterialien III, IV und V verglichen.

Material III repräsentiert ein typisches Mikrofüllermaterial (pyrogene Kieselsäure), Material IV enthält als Füllstoff ein Kieselsäuregranulat gemäß WO 81/01366 und Material V enthält einen typischen Makrofüller (Quarz)

|  | I | II | III | IV | V |
|---|---|---|---|---|---|
| Druckfestigkeit (N/mm²) | 300 | 290 | 250 | 280 | 240 |
| Biegefestigkeit (N/mm²) | 120 | 72 | 65 | 75 | 80 |
| Biegemodul (N/mm²) | 5800 | 2700 | 2600 | 3750 | 6800 |
| thermischer Ausdehnungskoeffizient (ppm/°C) | 28 | 64 | 70 | 46 | 27 |
| Wasseraufnahme (mg/cm²) | 0,42 | 0,81 | 0,70 | 0,25 | 0,30 |
| Polierbarkeit | ------ Hochglanz-------- | | | | matt |
| Abrasionsresistenz | ----- sehr gut---------- | | | | schwach |

Der Vergleich zeigt, daß das erfindungsgemäße dentale Füllungsmaterial die positiven physikalischen Eigenschaften der Makrofüller-Composites erreicht, deren Nachteile vermeidet, dafür jedoch die typischen positiven Eigenschaften eines Mikrofüller-Composites (Abrasionsresistenz, Hochglanzpolierbarkeit) aufweist.

Hinzu kommt eine sowohl die mikro- als auch die makrogefüllten Composites überragende Biegefestigkeit.

**Patentansprüche**

1. Verwendung eines oberflächenverkleinerten Kieselgels einer mittleren Korngröße von etwa 0,5 bis 50 µm, das dadurch hergestellt wurde, daß man ein übliches Kieselgel etwa der gleichen Korngröße mit größerer spezifischer Oberfläche einer thermischen Behandlung durch einen Glühprozess zwischen 500 und 1 200 °C und/oder einer chemisch-katalytischen Behandlung mit Säure unterwarf, als Füllstoff für Dentalmassen auf Basis polymerisierbarer Monomerer.

2. Verwendung nach Anspruch 1, worin das Kieselgel eine mittlere Korngröße von 1-20 µm aufweist.

3. Verwendung nach Anspruch 1 oder 2, wobei das Kieselgel zuerst der Behandlung mit Säure und anschließend dem Glühprozeß unterworfen worden war.

4. Verwendung nach einem der vorstehenden Ansprüche, wobei die polymerisierbaren Monomeren mono-, di- oder polyfunktionelle Ester der Acryl- oder Methacrylsäure sind.

5. Verwendung nach einem der vorstehenden Ansprüche zusammen mit anderen üblichen anorganischen und/oder organischen Füllstoffen.

6. Verwendung nach Anspruch 5, wobei die anderen anorganischen Füllstoffe Quarz, amorphes Siliciumdioxid, Gläser und/oder keramische oder mineralische Füllstoffe und die organischen Füllstoffe ungefüllte oder gefüllte Polymerisate sind.

7. Verwendung nach einem der vorstehenden Ansprüche, wobei die Füllstoffe oberflächenbehandelt, insbesondere silanisiert, sind.

8. Füllstoff für Dentalmassen auf Basis polymerisierbarer Monomerer, bestehend aus einem oberflächenverkleinerten Kieselgel einer mittleren Korngröße von etwa 0,5 bis 50 µm, das dadurch hergestellt wurde, daß man ein übliches Kieselgel etwa der gleichen Korngröße mit größerer spezifischer Oberfläche einer thermischen Behandlung durch einen Glühprozess zwischen 500 und 1 200 °C und/oder einer chemisch-katalytischen Behandlung mit Säure unterwarf.

9. Füllstoff nach Anspruch 8, dadurch gekennzeichnet, daß die mittlere Korngröße 1 bis 20 µm beträgt.

## Claims

1. Use of a surface area reduced silica gel of a mean grain size of about 0.5 to 50 µm, which has been prepared in that a conventional silica gel of approximately the same grain size with a larger specific surface is thermally treated by a roasting process between 500 and 1,200 °C and/or chemical-catalytically treated with an acid, as a filler for dental materials based on polymerizable monomers.

2. Use according to Claim 1, wherein the silica gel has a mean grain size of 1 to 20 µm.

3. Use according to Claim 1 or 2, wherein the silica gel was first treated with an acid followed by roasting.

4. Use according to one of the preceding claims, wherein the polymerizable monomers are mono-, di- or polyfunctional esters of acrylic acid or methacrylic acid.

5. Use according to one of the preceding claims together with other conventional inorganic and/or organic fillers.

6. Use according to claim 5, wherein the other inorganic fillers are quartz, amorphous silicon dioxide, glasses and/or ceramic or mineral fillers and the organic fillers are unfilled or filled polymers.

7. Use according to one of the preceding claims, wherein the fillers are surface treated, specifically silanized.

8. Filler for dental materials based on polymerizable monomers, consisting of a surface area reduced silica gel of a mean grain size of about 0.5 to 50 µm, which has been prepared in that a conventional silica gel of approximately the same grain size with a larger specific surface is thermally treated by a roasting process between 500 and 1,200 °C and/or chemical-catalytically treated with an acid.

9. Filler according to Claim 8, characterized in that the mean grain size is 1-20 µm.

## Revendications

1. Application d'un gel de silice à surface diminuée de granulométrie moyenne d'environ 0,5 à 50 µm, préparé en soumettant un gel de silice habituel d'environ la même granulométrie avec plus grande surface spécifique, à un traitement thermique par un procédé de grillage entre 500 et 1 200 °C et/ou à un traitement chimique-catalytique avec un acide, comme charge pour les masses dentaires à base de monomères polymérisables.

2. Application selon la revendication 1, où le gel de silice présente une granulométrie moyenne de 1-20 µm.

3. Application selon les revendications 1 et 2, où le gel de silice est tout d'abord soumis au traitement avec l'acide puis au procédé de grillage.

4. Application selon l'une des revendications précédentes, où les monomères polymérisables sont des esters mono-, di- ou polyfonctionnels de l'acide acrylique ou méthacrylique.

5. Application selon l'une des revendications précédentes avec d'autres charges inorganiques et/ou organiques habituelles.

6. Application selon la revendication 5, où les autres charges inorganiques sont le quartz, le dioxyde de silicium amorphe, les verres et/ou les charges céramiques ou minérales et les charges organiques sont des polymérisats non chargés ou chargés.

7. Application selon l'une des revendications précédentes, où les charges sont traitées à leur surface, en particulier silanisées.

8. Charge pour masses dentaires à base de monomères polymerisables, constituée d'un gel de silice à surface réduite d'une granulométrie moyenne d'environ 0,5 à 50 µm, préparée en soumettant un gel de silice habituel d'environ la même granulométrie et ayant une plus grande surface spécifique, à un traitement thermique par un procédé de grillage entre 500 et 1 200 °C et/ou à un traitement chimique/catalytique avec un acide.

9. Charge selon la revendication 8, caractérisée en ce que la granulométrie moyenne s'élève à 1 à 20 µm.